# EUROPEAN PATENT SPECIFICATION

(11) **EP 2 381 978 B1**
(45) Date of publication and mention of the grant of the patent: **12.07.2017**
(21) Application number: 09801361.8
(22) Date of filing: 23.12.2009
(51) Int. Cl.: A61M 5/142, H01Q 9/26

(54) **SPLIT RING RESONATOR ANTENNA ADAPTED FOR USE IN WIRELESSLY CONTROLLED MEDICAL DEVICE**
SPALTRING-RESONATOR-ANTENNE ZUR VERWENDUNG IN EINER DRAHTLOS GESTEUERTEN MEDIZINISCHEN VORRICHTUNG
ANTENNE À RÉSONATEUR À BAGUE FENDUE CONÇUE POUR UTILISATION DANS UN DISPOSITIF MÉDICAL COMMANDÉ SANS FIL

(30) Priority: 31.12.2008 US 141781 P
(43) Date of publication of application: 02.11.2011
(73) Proprietor: DEKA Products Limited Partnership, Manchester, NH 03101-1129 (US)
(72) Inventor: BLUMBERG, David, Epping NH 03042 (US); KAMEN, Dean, Bedford NH 03110 (US); GRAY, Larry, B., Merrimack NH 03054 (US)
(74) Representative: Greene, Simon Kenneth
(86) International application number: PCT/US2009/069491
(87) International publication number: WO 2010/078207

(56) References cited:
- EP-A1- 1 675 212
- DE-C1- 19 624 215
- FR-A1- 2 877 227
- KAROUI M S ET AL: "Study and design of a loop antenna for application of medical telemetry" INDUSTRIAL TECHNOLOGY, 2004. IEEE ICIT '04. 2004 IEEE INTERNATIONAL CO NFERENCE ON HAMMAMET, TUNSIA DEC. 8-10, 2004, PISCATAWAY, NJ, USA,IEEE LNKD- DOI:10.1109/ICIT.2004.1490804, vol. 3, 8 December 2004 (2004-12-08), pages 1589-1595, XP010822488 ISBN: 978-0-7803-8662-4
- YEKEH K ET AL: "Wireless Communications for Body Implanted Medical Device" MICROWAVE CONFERENCE, 2007. APMC 2007. ASIA-PACIFIC, IEEE, PISCATAWAY, NJ, USA, 11 December 2007 (2007-12-11), pages 1-4, XP031279795 ISBN: 978-1-4244-0748-4

## Description

### FIELD OF THE INVENTION

This application relates generally to split ring resonator antennas and more particularly to a split ring resonator antenna adapted for use in wirelessly controlled medical device

### BACKGROUND

Many potentially valuable medicines or compounds, including biologicals, are not orally active due to poor absorption, hepatic metabolism or other pharmacokinetic factors. Additionally, some therapeutic compounds, although they can be orally absorbed, are sometimes required to be administered so often it is difficult for a patient to maintain the desired schedule. In these cases, parenteral delivery is often employed or could be employed.

Effective parenteral routes of drug delivery, as well as other fluids and compounds, such as subcutaneous injection, intramuscular injection, and intravenous (IV) administration include puncture of the skin with a needle or stylet. Insulin is an example of a therapeutic fluid that is self-injected by millions of diabetic patients. Users of parenterally delivered drugs may benefit from a wearable device that would automatically deliver needed drugs/compounds over a period of time.

To this end, there have been efforts to design portable and wearable devices for the controlled release of therapeutics. Such devices are known to have a reservoir such as a cartridge, syringe, or bag, and to be electronically controlled. These devices suffer from a number of drawbacks including the malfunction rate. Reducing the size, weight and cost of these devices is also an ongoing challenge. Additionally, these devices often apply to the skin and pose the challenge of frequent re-location for application.

### SUMMARY OF THE INVENTION

In accordance with one aspect of the present invention an infusion pump assembly is disclosed. The infusion pump assembly includes a reservoir for receiving an infusible fluid, a pump assembly for pumping a quantity of infusible fluid from the reservoir to an exit, a first valve assembly configured to selectively isolate the pump assembly from the reservoir, a second valve assembly configured to selectively isolate the exit from the pumping assembly, and a split ring resonator antenna having a resonant frequency comprising a plurality of planar metallic layers.

Some embodiments of this aspect of the invention include one or more of the following. Wherein the split ring resonator antenna further includes an impedance matching circuit. Wherein the infusion pump assembly further includes at least one control unit and a transmitting and receiving base unit capable of powering the antenna. The split ring resonator antenna is coupled to the base unit and whereby the antenna wirelessly transmits and receives data from the at least one control unit, whereby the system minimizes the parasitic effects of dielectric materials in close proximity to a wearable radio frequency device. Wherein the apparatus further includes a signal processing component. wherein the signal processing component further comprising at least one filter. wherein the signal processing component further comprising at least one amplifier. wherein the signal processing component further comprising at least one switch. Wherein the infusion pump assembly further includes an external infusion set configured to deliver the infusible fluid to a user. Wherein the infusion pump assembly further includes a disposable housing assembly including the reservoir and a reusable housing assembly including a second portion of the fluid delivery system.

The details of one or more embodiments are set forth in the accompanying drawings and the description below. Other features and advantages will become apparent from the description, the drawings, and the claims.

### BRIEF DESCRIPTION OF THE DRAWINGS

FIG. 1 is a side view of an infusion pump assembly;
FIG. 2 is a perspective view of the infusion pump assembly of FIG. 1;
FIG. 3 is an exploded view of various components of the infusion pump assembly of FIG. 1;
FIG. 4 is a cross-sectional view of the disposable housing assembly of the infusion pump assembly of FIG. 1;
FIG. 5 is a perspective view of the infusion pump assembly of FIG. 1 showing an external infusion set;
FIG. 6 is an exemplary diagram of a split ring resonator antenna;
FIG. 7 is an exemplary diagram of a medical device configured to utilize a split ring resonator antenna;
FIG. 8 is an exemplary diagram of a split ring resonator antenna and transmission line from a medical infusion device;
FIG. 9 is a graph of the return loss of a split ring resonator antenna prior to contact with human skin;
FIG. 10 is a graph of the return loss of a split ring resonator antenna during contact with human skin;
FIG. 11 is an exemplary diagram of a split ring resonator antenna integrated into a device which operates within close proximity to dielectric material;
FIG. 12 is a diagram of the dimensions of the inner and outer portion of the exemplary embodiment;
FIG. 13 is a graph of the return loss of a non-split ring resonator antenna prior to contact with human skin;

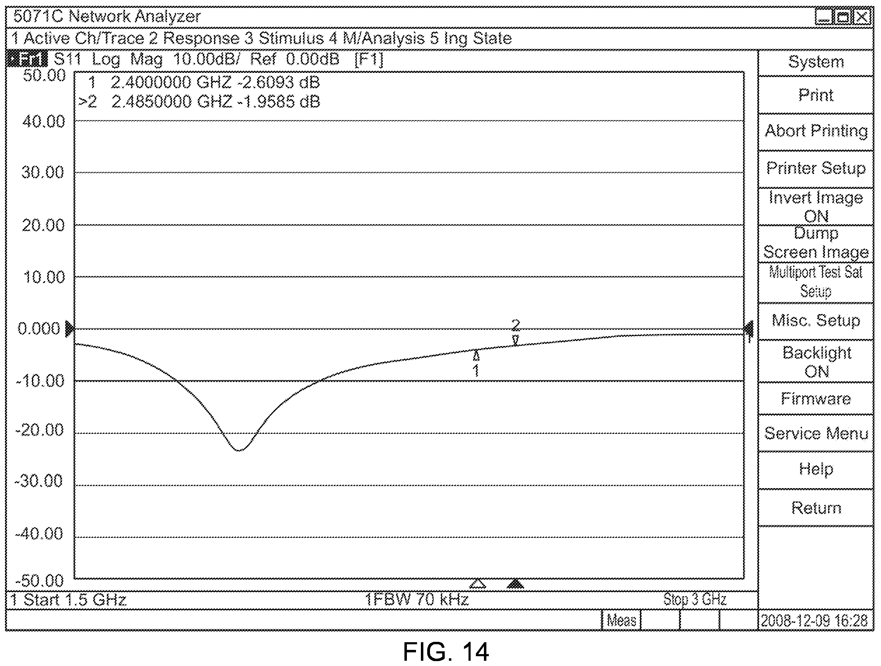

FIG. 14 is a graph of the return loss of a non-split ring resonator antenna during contact with human skin;
FIG. 15 is an exemplary diagram of another embodiment of the split ring resonator antenna and transmission line from a medical infusion device;
FIG. 16 is an outline drawings of the dimensions of the exemplary embodiment of the impedance matching circuit;
FIG. 17 is a connection illustration of the impedance matching circuit shown in FIG. 16;
FIG. 18 is a diagram of the dimensions of the inner and outer portion of the exemplary embodiment including an impedance matching circuit;
FIG. 19 is a schematic diagram of the impedance matching circuit shown in FIG. 16;
FIG. 20 is an outline drawings of the dimensions of one embodiment of the impedance matching circuit;
FIG. 21 is a connection illustration of the impedance matching circuit shown in FIG. 16; and
FIG. 22 is a schematic diagram of the impedance matching circuit shown in FIG. 16.
Like reference symbols in the various drawings indicate like elements.

### DETAILED DESCRIPTION OF THE PREFERRED EMBODIMENTS

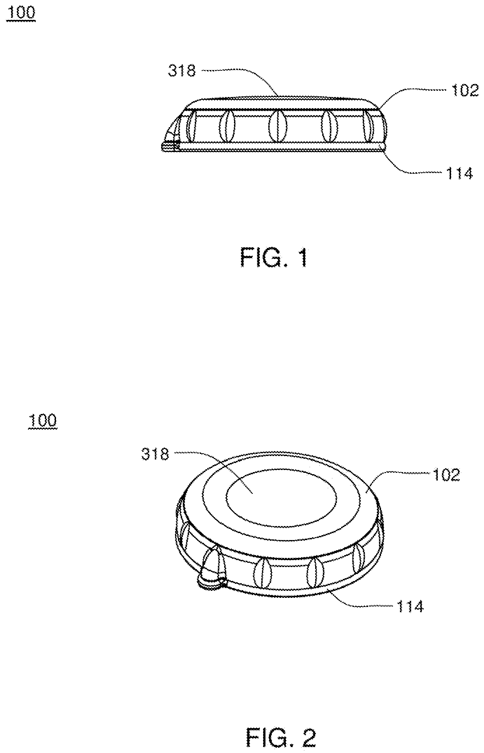

Referring to FIGS. 1-3, an infusion pump assembly 100 may include a reusable housing assembly 102. Reusable housing assembly 102 may be constructed from any suitable material, such as a hard or rigid plastic, that will resist compression. For example, use of durable materials and parts may improve quality and reduce costs by providing a reusable portion that lasts longer and is more durable, providing greater protection to components disposed therein.

Reusable housing assembly 102 may include mechanical control assembly 104 having a pump assembly 106 and at least one valve assembly 108. Reusable housing assembly 102 may also include electrical control assembly 110 configured to provide one or more control signals to mechanical control assembly 104 and effectuate the basal and/or bolus delivery of an infusible fluid to a user. Disposable housing assembly 114 may include valve assembly 108 which may be configured to control the flow of the infusible fluid through a fluid path. Reusable housing assembly 102 may also include pump assembly 106 which may be configured to pump the infusible fluid from the fluid path to the user.

Electrical control assembly 110 may monitor and control the amount of infusible fluid that has been and/or is being pumped. For example, electrical control assembly 110 may receive signals from volume sensor assembly 148 and calculate the amount of infusible fluid that has just been dispensed and determine, based upon the dosage required by the user, whether enough infusible fluid has been dispensed. If enough infusible fluid has not been dispensed, electrical control assembly 110 may determine that more infusible fluid should be pumped. Electrical control assembly 110 may provide the appropriate signal to mechanical control assembly 104 so that any additional necessary dosage may be pumped or electrical control assembly 110 may provide the appropriate signal to mechanical control assembly 104 so that the additional dosage may be dispensed with the next dosage. Alternatively, if too much infusible fluid has been dispensed, electrical control assembly 110 may provide the appropriate signal to mechanical control assembly 104 so that less infusible fluid may be dispensed in the next dosage.

Mechanical control assembly 104 may include at least one shape-memory actuator 112. Pump assembly 106 and/or valve assembly 108 of mechanical control assembly 104 may be actuated by at least one shape-memory actuator, e.g., shape-memory actuator 112, which may be a shape-memory wire in wire or spring configuration. Shape memory actuator 112 may be operably connected to and activated by electrical control assembly 110, which may control the timing and the amount of heat and/or electrical energy used to actuate mechanical control assembly 104. Shape memory actuator 112 may be, for example, a conductive shape-memory alloy wire that changes shape with temperature. The temperature of shape-memory actuator 112 may be changed with a heater, or more conveniently, by application of electrical energy. Shape memory actuator 112 may be a shape memory wire constructed of nickel/titanium alloy, such as NITINOL™ or FLEXINOL®.

Infusion pump assembly 100 may include a volume sensor assembly 148 configured to monitor the amount of fluid infused by infusion pump assembly 100. For example, volume sensor assembly 148 may employ, for example, acoustic volume sensing. Acoustic volume measurement technology is the subject of U.S. Patent Nos. 5,575,310 and 5,755,683 assigned to DEKA Products Limited Partnership, as well as U.S. patent application Publication Nos. US 2007/0228071 A1, US 2007/0219496 A1, US 2007/0219480 A1, US 2007/0219597 A1, the entire disclosures of all of which are incorporated herein by reference. Other alternative techniques for measuring fluid flow may also be used; for example, Doppler-based methods; the use of Hall-effect sensors in combination with a vane or flapper valve; the use of a strain beam (for example, related to a flexible member over a fluid reservoir to sense deflection of the flexible member); the use of capacitive sensing with plates; or thermal time of flight methods. One such alternative technique is disclosed in U.S. Patent application Serial No. 11/704,899, entitled Fluid Delivery Systems and Methods, filed 09 February 2007, the entire disclosure of which is incorporated herein by reference. Infusion pump assembly 100 may be configured so that the volume measurements produced by volume sensor assembly 148 may be used to control, through a feedback loop, the amount of infusible fluid that is infused into the user.

Infusion pump assembly 100 may further include a disposable housing assembly 114. For example, disposable housing assembly 114 may be configured for a single use or for use for a specified period of time, e.g., three days or any other amount of time. Disposable housing assembly 114 may be configured such that any components in infusion pump assembly 100 that come in contact with the infusible fluid are disposed on and/or within disposable housing assembly 114. For example, a fluid path or channel including a reservoir, may be positioned within disposable housing assembly 114 and may be configured for a single use or for a specified number of uses before disposal. The disposable nature of disposable housing assembly 114 may improve sanitation of infusion pump assembly 100.

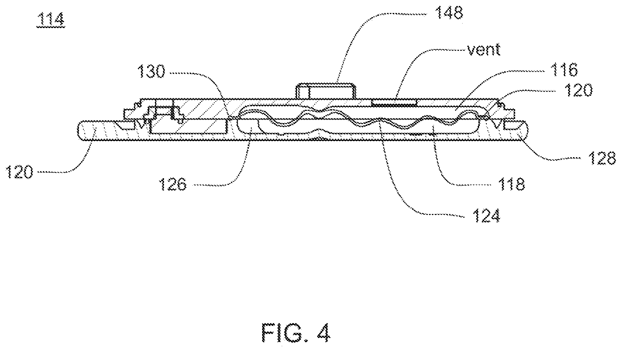

Referring also to FIG. 4, disposable housing assembly 114 may be configured to releasably engage reusable housing assembly 102, and includes a cavity 116 that has a reservoir 118 for receiving an infusible fluid (not shown), e.g., insulin. Such releasable engagement may be accomplished by a screw-on, a twist-lock or a compression fit configuration, for example. Disposable housing assembly 114 and/or reusable housing assembly 102 may include an alignment assembly configured to assist in aligning disposable housing assembly 114 and reusable housing assembly 102 for engagement in a specific orientation. Similarly, base nub 120 and top nub 122 may be used as indicators of alignment and complete engagement.

Cavity 116 may be at least partially formed by and integral to disposable housing assembly 114. Cavity 116 may include a membrane assembly 124 for at least partially defining reservoir 118. Reservoir 118 may be further defined by disposable housing assembly 114, e.g., by a recess 126 formed in base portion 128 of disposable housing assembly 114. For example, membrane assembly 124 may be disposed over recess 126 and attached to base portion 128, thereby forming reservoir 118. Membrane assembly 124 may be attached to base portion 128 by conventional means, such as gluing, heat sealing, and/or compression fitting, such that a seal 130 is formed between membrane assembly 124 and base portion 128. Membrane assembly 124 may be flexible and the space formed between membrane assembly 124 and recess 126 in base portion 128 may define reservoir 118. Reservoir 118 may be non-pressurized and in fluid communication with a fluid path (not shown). Membrane assembly 124 may be at least partially collapsible and cavity 116 may include a vent assembly, thereby advantageously preventing the buildup of a vacuum in reservoir 118 as the infusible fluid is delivered from reservoir 118 to the fluid path. In a preferred embodiment, membrane assembly 124 is fully collapsible, thus allowing for the complete delivery of the infusible fluid. Cavity 116 may be configured to provide sufficient space to ensure there is always some air space even when reservoir 118 is filled with infusible fluid.

The membranes and reservoirs described herein may be made from materials including but not limited to silicone, NITRILE, and any other material having desired resilience and properties for functioning as described herein. Additionally, other structures could serve the same purpose.

The use of a partially collapsible non pressurized reservoir may advantageously prevent the buildup of air in the reservoir as the fluid in the reservoir is depleted. Air buildup in a vented reservoir could prevent fluid egress from the reservoir, especially if the system is tilted so that an air pocket intervenes between the fluid contained in the reservoir and the septum of the reservoir. Tilting of the system is expected during normal operation as a wearable device.

Reservoir 118 may be conveniently sized to hold an insulin supply sufficient for delivery over one or more days. For example, reservoir 118 may hold about 1.00 to 3.00 ml of insulin. A 3.00 ml insulin reservoir may correspond to approximately a three day supply for about 90% of potential users. In other embodiments, reservoir 118 may be any size or shape and may be adapted to hold any amount of insulin or other infusible fluid. In some embodiments, the size and shape of cavity 116 and reservoir 118 is related to the type of infusible fluid that cavity 116 and reservoir 118 are adapted to hold.

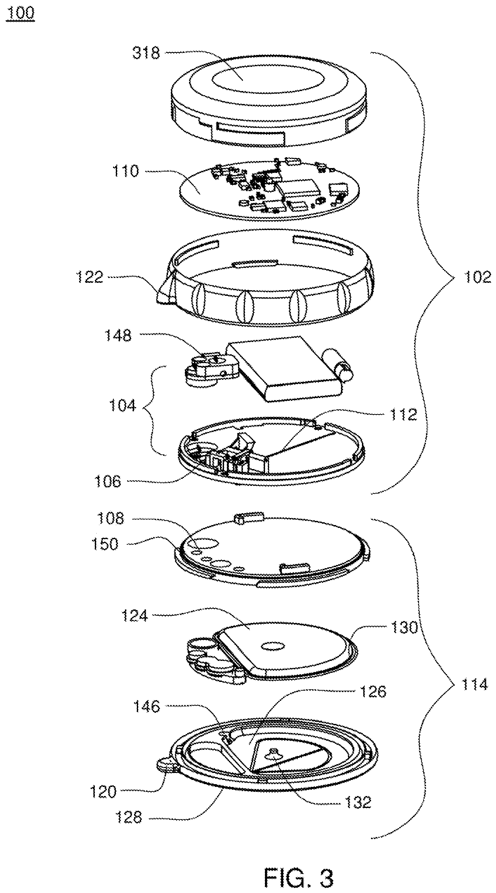

Disposable housing assembly 114 may include a support member 132 (FIG. 3) configured to prevent accidental compression of reservoir 118. Compression of reservoir 118 may result in an unintentional dosage of infusible fluid being forced through the fluid path to the user. In a preferred embodiment, reusable housing assembly 102 and disposable housing assembly 114 may be constructed of a rigid material that is not easily compressible. However, as an added precaution, support member 132 may be included within disposable housing assembly 114 to prevent compression of infusion pump assembly 100 and cavity 116 therein. Support member 132 may be a rigid projection from base portion 128. For example, support member 132 may be disposed within cavity 116 and may prevent compression of reservoir 118.

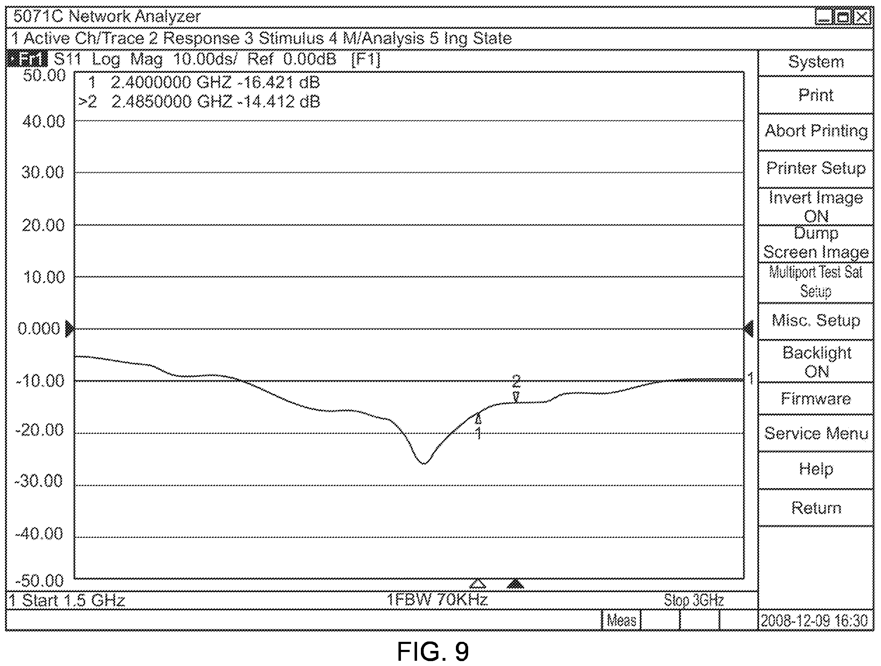

As discussed above, cavity 116 may be configured to provide sufficient space to ensure there is always some air space even when reservoir 118 is filled with infusible fluid. Accordingly, in the event that infusion pump assembly 100 is accidentally compressed, the infusible fluid may not be forced through cannula assembly 136 (e.g., shown in FIG. 9).

Cavity 116 may include a septum assembly 146 (FIG. 3) configured to allow reservoir 118 to be filled with the infusible fluid. Septum assembly 146 may be a conventional septum made from rubber or plastic and have a one-way fluid valve configured to allow a user to fill reservoir 118 from a syringe or other filling device. In some embodiments, septum 146 may be located on the top of membrane assembly 124. In these embodiments, cavity 116 may include a support structure (e.g., support member 132 in FIG. 3) for supporting the area about the back side of the septum so as to maintain the integrity of the septum seal when a needle is introducing infusible fluid into cavity 116. The support structure may be configured to support the septum while still allowing the introduction of the needle for introducing infusible fluid into cavity 116.

Infusion pump assembly 100 may include an overfill prevention assembly (not shown) that may e.g., protrude into cavity 116 and may e.g., prevent the overfilling of reservoir 118.

In some embodiments, reservoir 118 may be configured to be filled a plurality of times. For example, reservoir 118 may be refillable through septum assembly 146. As infusible fluid may be dispensed to a user, electronic control assembly 110 may monitor the fluid level of the infusible fluid in reservoir 118. When the fluid level reaches a low point, electronic control assembly 110 may provide a signal, such as a light or a vibration, to the user that reservoir 118 needs to be refilled. A syringe, or other filling device, may be used to fill reservoir 118 through septum 146.

Reservoir 118 may be configured to be filled a single time. For example, a refill prevention assembly (not shown) may be utilized to prevent the refilling of reservoir 118, such that disposable housing assembly 114 may only be used once. The refill prevention assembly (not shown) may be a mechanical device or an electro-mechanical device. For example, insertion of a syringe into septum assembly 146 for filling reservoir 118 may trigger a shutter to close over septum 146 after a single filling, thus preventing future access to septum 146. Similarly, a sensor may indicate to electronic control assembly 110 that reservoir 118 has been filled once and may trigger a shutter to close over septum 146 after a single filling, thus preventing future access to septum 146. Other means of preventing refilling may be utilized and are considered to be within the scope of this disclosure.

As discussed above, disposable housing assembly 114 may include septum assembly 146 that may be configured to allow reservoir 118 to be filled with the infusible fluid. Septum assembly 146 may be a conventional septum made from rubber or any other material that may function as a septum, or, in other embodiments, septum assembly 146 may be, but is not limited to, a plastic, or other material, one-way fluid valve. In various embodiments, including the exemplary embodiment, septum assembly 146 is configured to allow a user to fill reservoir 118 from a syringe or other filling device. Disposable housing assembly 114 may include a septum access assembly that may be configured to limit the number of times that the user may refill reservoir 118.

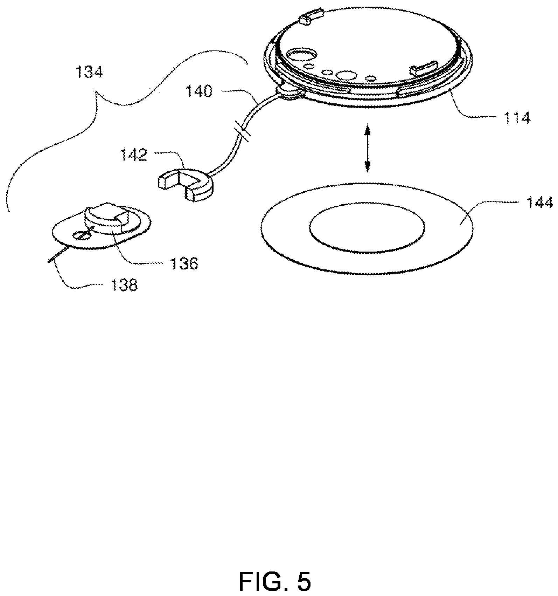

Referring also to FIG. 5, infusion pump assembly 100 may include an external infusion set 134 configured to deliver the infusible fluid to a user. External infusion set 134 may be in fluid communication with cavity 118, e.g. by way of the fluid path. External infusion set 134 may be disposed adjacent to infusion pump assembly 100. Alternatively, external infusion set 134 may be configured for application remote from infusion pump assembly 100, as discussed in greater detail below. External infusion set 134 may include a cannula assembly 136, which may include a needle or a disposable cannula 138, and tubing assembly 140. Tubing assembly 140 may be in fluid communication with reservoir 118, for example, by way of the fluid path, and with cannula assembly 138 for example, either directly or by way of a cannula interface 142.

External infusion set 134 may be a tethered infusion set, as discussed above regarding application remote from infusion pump assembly 100. For example, external infusion set 134 may be in fluid communication with infusion pump assembly 100 through tubing assembly 140, which may be of any length desired by the user (e.g., 3-18 inches). Though infusion pump assembly 100 may be worn on the skin of a user with the use of adhesive patch 144, the length of tubing assembly 140 may enable the user to alternatively wear infusion pump assembly 100 in a pocket. This may be beneficial to users whose skin is easily irritated by application of adhesive patch 144. Similarly, wearing and/or securing infusion pump assembly 100 in a pocket may be preferable for users engaged in physical activity.

In addition to / as an alternative to adhesive patch 144, a hook and loop fastener system (e.g. such as hook and loop fastener systems offered by Velcro USA Inc. of Manchester, NH) may be utilized to allow for easy attachment / removal of an infusion pump assembly (e.g., infusion pump assembly 100) from the user. Accordingly, adhesive patch 144 may be attached to the skin of the user and may include an outward facing hook or loop surface. Additionally, the lower surface of disposable housing assembly 114 may include a complementary hook or loop surface. Depending upon the separation resistance of the particular type of hook and loop fastener system employed, it may be possible for the strength of the hook and loop connection to be stronger than the strength of the adhesive to skin connection. Accordingly, various hook and loop surface patterns may be utilized to regulate the strength of the hook and loop connection.

In the exemplary embodiments, the infusion pump assembly may be wirelessly controlled by a remote control device. In the exemplary embodiments, a split ring resonator antenna may be used for wireless communication between the infusion pump assembly and the remote control device (or other remote device). The term "wirelessly controlled" refers to any device that may receive input, instructions, data, or other, wirelessly. Further, a wirelessly controlled insulin pump refers to any insulin pump that may wirelessly transmit and/or receive data from another device. Thus, for example, an insulin pump may both receive instructions via direct input by a user and may receive instructions wirelessly from a remote controller.

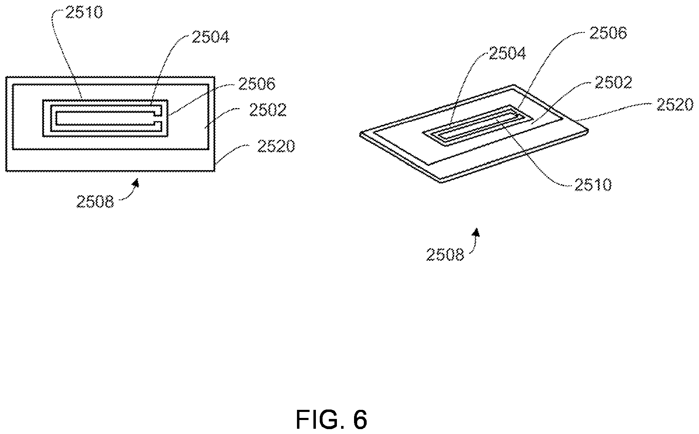

Referring to FIG. 6 an exemplary embodiment of a split ring resonator antenna adapted for use in a wirelessly controlled medical device, and is used in the exemplary embodiment of the infusion pump assembly, includes at least one split ring resonator antenna (hereinafter "SRR antenna") 2508, a wearable electric circuit, such as a wirelessly controlled medical infusion apparatus (hereinafter "infusion apparatus") 2514, capable of powering the antenna, and a control unit 2522.

In various embodiments, a SRR antenna 2508 may reside on the surface of a nonconducting substrate base 2520, allowing a metallic layer (or layers) to resonate at a predetermined frequency. The substrate base 2520 may be composed of standard printed circuit board material such as Flame Retardant 2 (FR-2), FR-3, FR-4, FR-5, FR-6, G-10, CEM-1, CEM-2, CEM-3, CEM-4, CEM-5, Polyimide, Teflon, ceramics, or flexible Mylar. The metallic resonating bodies comprising a SRR antenna 2508 may be made of two rectangular metallic layers 2502, 2504, made of, for example, platinum, iridium, copper, nickel, stainless steel, silver or other conducting materials. In other various embodiments, a SRR antenna 2508 may contain only one metallic resonating body.

In the exemplary embodiment, a gold-plated copper outer layer 2502, surrounds, without physically contacting, a gold-plated copper inner ring 2504. That is, the inner ring 2504 resides in the cavity 2510 (or aperture) formed by the outer layer 2502. The inner ring 2504 may contain a gap, or split 2506, along its surface completely severing the material to form an incomplete ring shape. Both metallic resonating bodies 2502, 2504 may reside on the same planar surface of the substrate base 2520. In such a configuration, the outer layer 2502 may by driven via a transmission line 2512 coupled to the outer layer 2502, for example. Additionally, in various other embodiments, a transmission line 2512 may be coupled to the inner ring 2504.

Antenna design software, such as AWR Microwave Office, capable of simulating electromagnetic geometries, such as, antenna performance, may significantly decrease the time required to produce satisfactory dimensions compared to physically fabricating and testing antennas. Accordingly, with aid of such software, the SRR antenna 2508 may be designed such that the geometric dimensions of the resonant bodies 2502, 2504 facilitate an operational frequency of the 2.4GHz ISM Band. FIG. 132 depicts the exemplary dimensions of the inner ring 2504 and outer layer 2502, and the positioning of the cavity 2510 in which the inner ring 2504 resides. The distance in between the outer layer 2502 and the inner ring 2504 is a constant 0.005 inches along the perimeter of the cavity 2510. However, in other embodiments, the distance between the outer layer and the inner ring may vary and in some embodiments, the operational frequency may vary.

In various embodiments, a SRR antenna 2508 may have dimensions such that it could be categorized as electrically small, that is, the greatest dimension of the antenna being less than one wavelength at operational frequency.

In various other embodiments, a SRR antenna 2508 may be composed of one or more alternatively-shaped metallic outer layers, such as circular, pentagonal, octagonal, or hexagonal, surrounding one or more metallic inner layers of similar shape. Further, in various other embodiments, one or more metallic layers of a SRR antenna 2508 may contain gaps in the material.

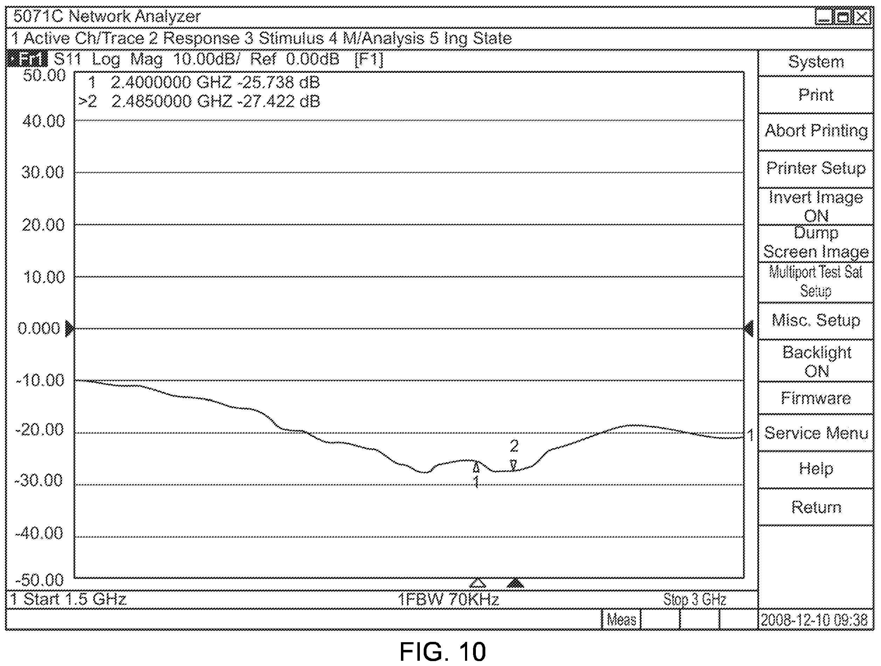

Referring to FIG. 9, a SRR antenna 2508 having the exemplary geometry exhibits acceptable return loss and frequency values when placed in contact with human skin. As shown in FIG. 130, focusing on the band of interest denoted by markers 1 and 2 on the graph, return loss prior to contact with human skin is near -15 dB while monitoring a frequency band centered around the 2.44 GHz ISM Band. Return loss during contact with human skin, as shown in FIG. 10, remains a suitable value near -25 dB at the same frequency, yielding approximately 97% transmission power.

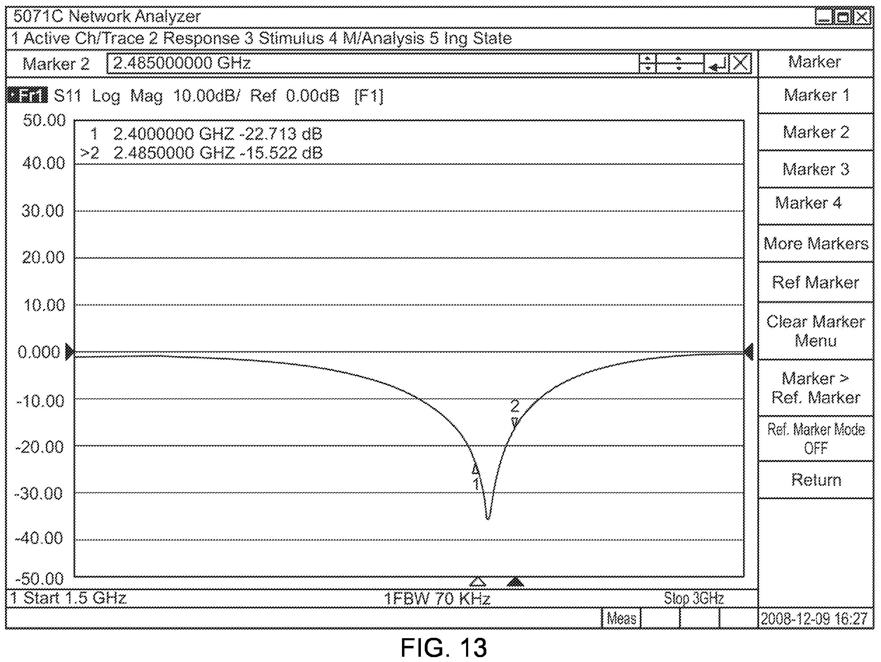

These results are favorable especially as compared with a non-split ring resonator antenna type, such as the Inverted-F. Return loss of an Inverted-F antenna may exhibit a difference when the antenna contacts human skin, resulting in a low percentage of power transmitted outward from the antenna. By way of example, as shown in FIG. 13, and again focusing on the band of interest denoted by markers 1 and 2 on the graph, return loss of an Inverted-F antenna prior to contact with human skin is near -25 dB at a frequency centered around 2.44 GHz. Return loss during contact with human skin is nearly -2 dB at the same frequency, yielding approximately 37% power transmission.

### Integration with a Wireless Medical Device

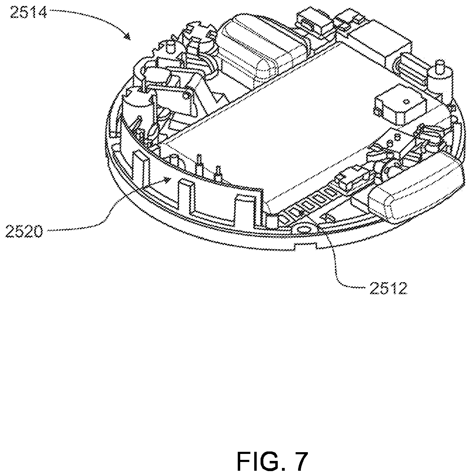

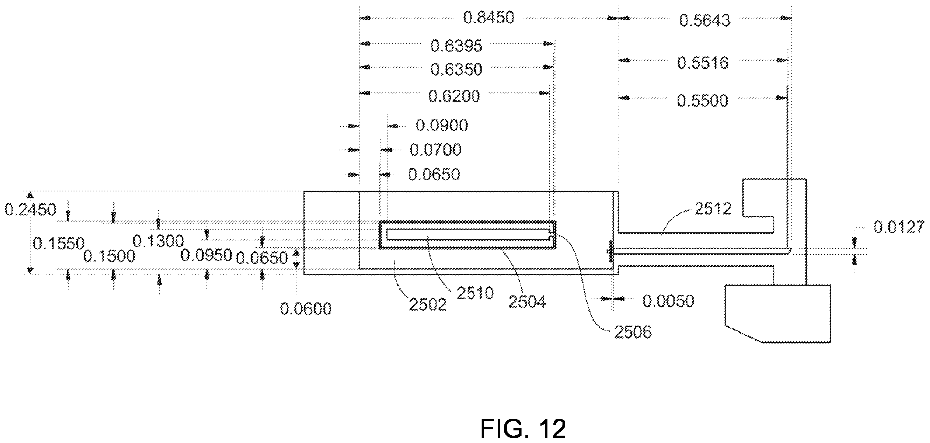

In the exemplary embodiment, referring to FIG. 12 and FIG. 7, one application of a SRR antenna 2508 may be integration into a wearable infusion apparatus 2514 capable of delivering fluid medication to a user/patient 2524. In such an application, the safety of the user/patient is dependent on fluid operation between these electrical components, thus reliable wireless transmission to and from a control unit 2522 is of great importance.

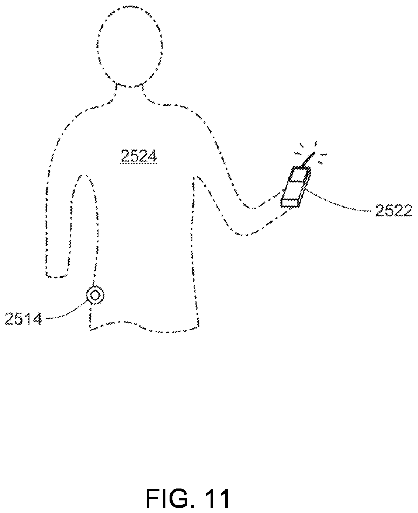

An infusion apparatus 2514 may be worn directly on the human body. By way of example, such a device may be attached on or above the hip joint in direct contact with human skin, placing the SRR antenna 2508 at risk of unintended dielectric loading causing a frequency shift in electrical operation. However, in such an application, electrical characteristics of the SRR antenna 2508 which allow it to be less sensitive to surrounding materials are beneficial in reducing or eliminating degradation to the performance. A controlling component, such as a control unit 2522 (generally shown in FIG. 11), may be paired with an infusion apparatus 2514, and may be designed to transmit and receive wireless signals to and from the infusion apparatus 2514 at a predetermined frequency band, which, in the exemplary embodiment, is the 2.4GHz Industrial Scientific and Medical Band ("ISM band"). In the exemplary embodiment, the control unit 2522 serves as the main user interface through which a patient or third party may manage insulin delivery. In other embodiments, infusion apparatus 2514 may utilize a SRR antenna 2508 to communicate with one or more control units 2522.

In various embodiments, a number of different wireless communication protocols may be used in conjunction with the SRR antenna 2508, as the protocol and data types to be transferred are independent of the electrical characteristics of the antenna. However, in the exemplary embodiment, a bi-directional master/slave means of communication organizes the data transfer through the SRR antenna 2508. The control unit 2522 may act as the master by periodically polling the infusion apparatus 2514, or slave, for information. In the exemplary embodiment, only when the slave is polled, the slave may send signals to the control unit 2522 only when the slave is polled. However, in other embodiments, the slave may send signals before being polled. Signals sent by way of this system may include, but are not limited to, control, alarm, status, patient treatment profile, treatment logs, channel selection and negotiation, handshaking, encryption, and check-sum. In some embodiments, transmission through the SRR antenna 2508 may also be halted during certain infusion operations as an added precaution against electrical disruption of administration of insulin to the patient.

In the exemplary embodiment, the SRR antenna 2508 may be coupled to electrical source circuitry via one or more pins 2516 on a transmission line 2512. In various other embodiments a transmission line may comprise a wire, pairs of wire, or other controlled impedance methods providing a signal path to the SRR antenna 2508. The transmission line 2512 may reside on the surface of the substrate base 2520 and may be composed of the same material as the SRR antenna 2508, such as gold-plated copper. Additionally, a ground plane may be attached to the surface of the substrate base opposite the transmission line 2512.

The electrical circuitry coupled to the SRR antenna 2508 may apply an RF signal to the end of the transmission line 2512 nearest the circuitry, creating an electromagnetic field throughout, and propagating from, the SRR antenna 2508. The electrical circuitry coupled to the SRR antenna 2508 facilitates resonance at a predetermined frequency band, which, in the exemplary embodiment, is the 2.4GHz ISM band. Preferably, transmission line 2512 and SRR antenna 2508 both have impedances of 50 Ohms to simplify circuit simulation and characterization. However, in other various embodiments, the transmission line and SRR antenna 2508 may have other impendence values, or a different resonating frequency.

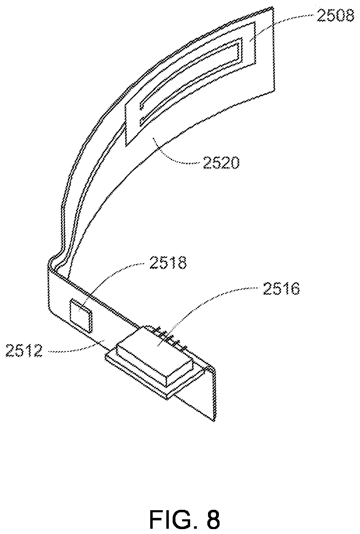

Referring to FIG. 8, a signal processing component(s) 2518, such as, a filter, amplifier, or switch, may be integrated into the transmission line 2512, or at some point between the signal source connection pins 2516 and the SRR antenna 2508. In the exemplary embodiment, the signal processing component 2518 is a band-pass filter to facilitate desired signal processing, such as, allowing only the exemplary frequency band to be transmitted to the antenna, and rejecting frequencies outside that range. In the exemplary embodiment, a Combline band-pass filter 2518 may be included in the transmission line 2512 between the antenna and the signal source. However in other embodiments, any other signal processing device, for example, but not limited to, filters, amplifiers, or any other signal processing devices known in the art.

In various embodiments, a SRR antenna 2508 may be composed of metallic bodies capable of resonating on a flexible or rigid substrate. As shown in FIG. 7 and FIG. 8, the exemplary embodiment incorporates a curved SRR antenna on a flexible Polyimide substrate 2520. Polyimide may be the exemplary material because it tends to be more flexible than alternative substrates. This configuration may allow for simplified integration into circular-shaped devices (such as a wirelessly controlled medical infusion apparatus 2514), devices with irregular-shaped external housing, or devices in which saving space is paramount.

In various embodiments, both control unit 2522 and base unit 2514 may incorporate a SRR antenna 2508. This configuration may prove beneficial where the control unit is meant to be handheld, in close proximity to human skin, or is likely to be in close proximity to a varying number of materials with varying dielectric constants.

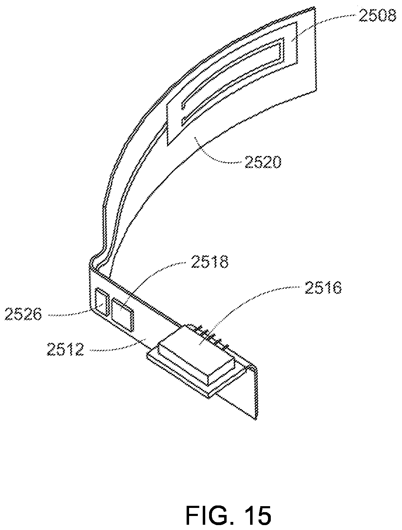

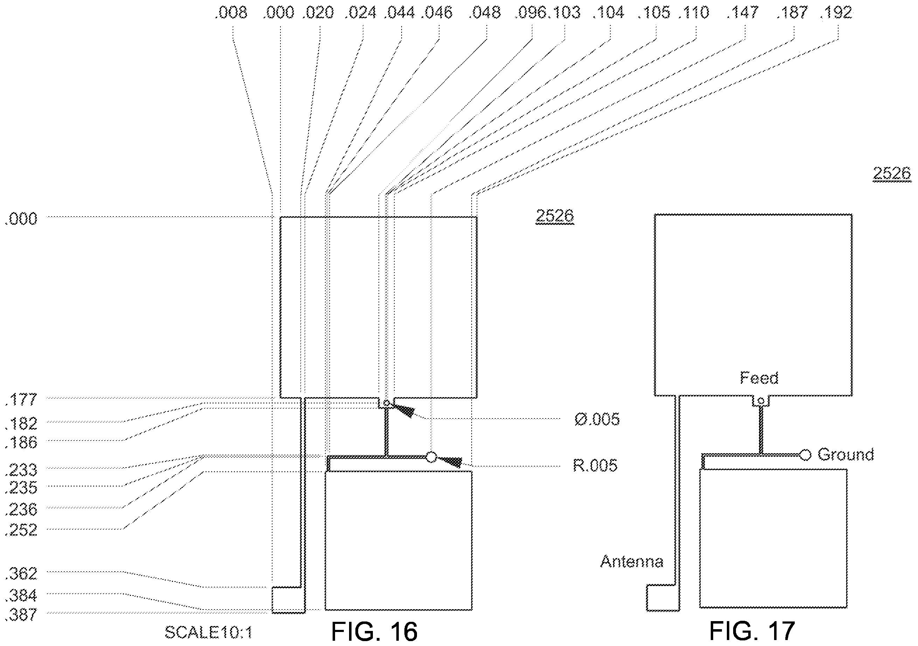

Referring now to FIG. 15, in various embodiments, an impedance matching circuit 2526 may be integrated into the transmission line 2512. In some embodiments, the impedance matching circuit 2526 may be integrated between the signal processing component(s) 2518 and the SRR antenna 2508. The embodiments including an impedance matching circuit 2526 eliminate the need for a ground plane for the SRR antenna 2508. Referring now to FIGS. 135A and 135B, outline drawings, showing the dimensions and the mechanical configuration, respectively, for one embodiment of the impedance matching circuit 2526 are shown. The dimensions shown in these FIGS. are for one embodiment of the impedance matching circuit 2526. However, in other embodiments, the dimensions may be different than those shown in FIGS. 16 and 17. The dimensions used may depend on one or more factors, including, but not limited to, the thickness of the substrate 2512 and/or the material/ dielectric constant of the substrate 2512. For example, where a substrate has a lower dielectric constant, the geometry of the impedance matching circuit 2526 may be larger, and where the dielectric constant is higher, the geometry of the impedance matching circuit 2526 may be smaller. In some embodiments, the size of the device in which the SRR antenna 2508 may be used may influence the substrate selected, i.e., the size of the device may influence the design of the impedance matching circuit 2526. For example, in some embodiments, the thickness of the substrate may influence the design, and in some embodiments, the shape of the substrate may change the design.

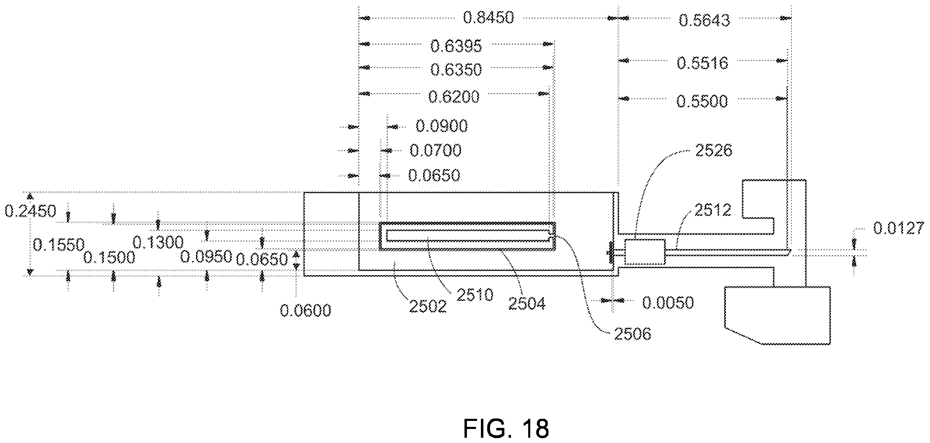

Referring now to FIG. 18, a diagram of the dimensions of the inner and outer portion of the exemplary embodiment of the SRR antenna, including an impedance matching circuit, is shown. The dimensions shown in FIGS. 132 and 136 are for one embodiment of the SRR antenna design. However, in other embodiments, the dimensions may be different than those shown in FIGS. 12 and 18.

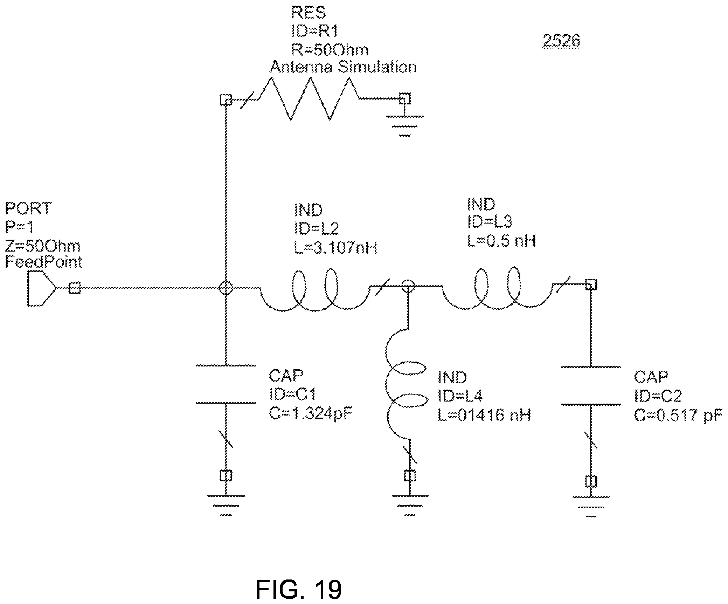

Referring now to FIG. 19, a schematic of one embodiment of the matching circuit, as shown in FIGS. 135A and 135B, is shown. The component values shown are representative of the geometry of the impendence matching circuit 2526 as shown in FIGS. 135A and 135B. The component values may vary with the impendence matching circuit 2526 design and/or geometry.

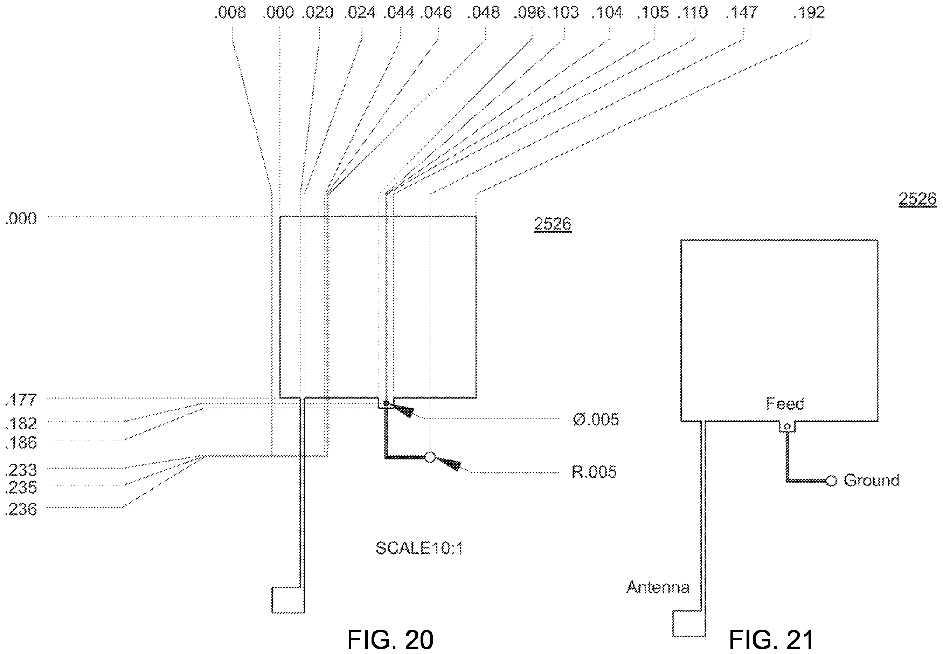

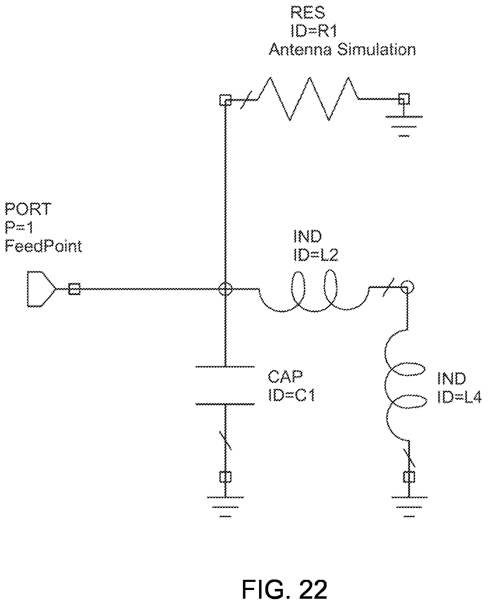

Referring now to FIGS. 20 and 21, another embodiment of the impedance matching circuit 2526 is shown. Referring now to FIG. 22, a schematic of one embodiment of the matching circuit, as shown in FIGS. 20 and 21, is shown. The component values shown are representative of the geometry of the impendence matching circuit 2526 as shown in FIGS. 20 and 21. The component values may vary with the impendence matching circuit 2526 design and/or geometry. In various embodiments, the design of the impendence matching circuit 2526 may vary.

In various other embodiments, a SRR antenna 2508 may be integrated into a human or animal limb replacement. As prosthetic limbs are becoming more sophisticated the electrical systems developed to control and simulate muscle movements require much more wiring and data transfer among subsystems. Wireless data transfer within a prosthetic limb may reduce weight through reduced physical wiring, conserve space, and allow greater freedom of movement. However, common antennas in such a system may be susceptible to dielectric loading. Similar to the previously mentioned benefits of integrating a SRR antenna 2508 into a wirelessly controlled medical infusion apparatus, a prosthetic limb, such as a robotic arm, may also come into contact with human skin or other dielectric materials and benefit from the reduction of electrical disturbances associated with such an antenna. In other various embodiments, the SRR antenna 2508 may be integrated into any device comprised of the electrical components capable of powering and transmitting/receiving data to an antenna and susceptible to electrical disturbances associated with proximity to dielectric materials.

In various embodiments, a SRR antenna 2508 may be integrated into a configuration of medical components in which one or more implantable medical devices, operating within the human body, communicate wirelessly to a handheld, body-mounted, or remote control unit. In certain embodiments, both body-mounted and in-body wireless devices may utilize a SRR antenna 2508 for wireless communication. Additionally, one or more of the components utilizing a SRR antenna 2508 may be completely surrounded by human skin, tissue or other dielectric material. By way of example, such a configuration may be used in conjunction with a heart monitoring/control system where stability and consistency of wireless data transmission are of fundamental concern.

In various other embodiments, a SRR antenna 2508 may be integrated into the embodiments of the infusion pump assembly. In some embodiments, the SRR antenna 2508 may be integrated into a configuration of medical components in which one or more electrical sensors positioned on, or attached to, the human body wirelessly communicate to a remote transceiving unit. By way of example, a plurality of electrodes positioned on the body may be coupled to a wireless unit employing a SRR antenna 2508 for wireless transmission to a remotely located electrocardiogram machine. By way of further example, a wireless temperature sensor in contact with human skin may employ SRR antenna 2508 for wireless communication to a controller unit for temperature regulation of the room in which the sensor resides.

## Claims

1. An infusion pump assembly
comprising:
a reservoir (118) for receiving an infusible fluid;
a pump assembly (106) for pumping a quantity of infusible fluid from the reservoir to an exit;
a first valve assembly (108) configured to selectively isolate the pump assembly from the reservoir;
a second valve assembly (108) configured to selectively isolate the exit from the pump assembly; and
a split ring resonator antenna (2508) having a resonant frequency comprising a plurality of planar metallic layers,
the split ring resonator antenna comprising an outer ring (2502), an inner ring (2504), wherein the inner ring (2504) is surrounded by the outer ring (2502) without physically contacting the outer ring, the inner ring having a split (2506) and
an impedance matching circuit (2526), wherein the impedance matching circuit is integrated into a transmission line.

2. The infusion pump assembly of claim 1 wherein the infusion pump assembly further comprising:
at least one control unit (2522); and
a transmitting and receiving base unit (2514) capable of powering the antenna, wherein the split ring resonator antenna is coupled to the base unit and whereby the antenna wirelessly transmits and receives data from the at least one control unit, whereby the system minimizes the parasitic effects of dielectric materials in close proximity to a wearable radio frequency device.

3. The infusion pump assembly of claim 2 further comprising a signal processing component (2518).

4. The infusion pump assembly of claim 3, wherein the impedance matching circuit (2526) is integrated adjacent to the signal processing component (2518).

5. The infusion pump assembly of claim 4, the signal processing component (2518) further comprising at least one filter.

6. The infusion pump assembly of claim 4, the signal processing component (2518) further comprising at least one amplifier.

7. The infusion pump assembly of claim 4, the signal processing component (2518) further comprising at least one switch.

8. The infusion pump assembly of claim 2 further comprising an external infusion set (134) configured to deliver the infusible fluid to a user.

9. The infusion pump assembly of claim 1 or 8 further comprising:
a disposable housing assembly (114) including a first portion of a fluid delivery system and the reservoir; and
a reusable housing assembly (102) including a second portion of the fluid delivery system.

10. The system of claim 1 wherein the signal processing component further comprising at least one filter.

11. The system of claim 1 wherein the signal processing component further comprising at least one amplifier.

12. The system of claim 1 wherein the signal processing component further comprising at least one switch.

## Patentansprüche

1. Infusionspumpenbaugruppe,
aufweisend:
einen Behälter (118) zum Aufnehmen eines Infusionsfluids,
eine Pumpenbaugruppe (106) zum Pumpen einer Menge Infusionsfluid vom Behälter zu einem Ausgang;
eine erste Ventilbaugruppe (108), die gestaltet ist, um die Pumpenbaugruppe selektiv von dem Behälter zu isolieren,
eine zweite Ventilbaugruppe (108), die gestaltet ist, um den Ausgang selektiv von der Pumpenbaugruppe zu isolieren, und
eine Spaltringresonatorantenne (2508) mit einer Resonanzfrequenz, aufweisend eine Vielzahl von ebenen metallischen Schichten,
wobei die Spaltringresonatorantenne einen Außenring (2502) und einen Innenring (2504) aufweist,
wobei der Innenring(2504) vom Außenring (2502) umgeben ist, ohne den Außenring physisch zu berühren, wobei der Innenring einen Spalt (2506) hat, und
eine Impedanzanpassungsschaltung (2526), wobei die Impedanzanpassungsschaltung in eine Übertragungsleitung integriert wird.

2. Infusionspumpenbaugruppe nach Anspruch 1, wobei die Infusionspumpenbaugruppe ferner Folgendes aufweist:
mindestens eine Steuerungseinheit (2522) und
eine Übertragungs- und Empfangs-Basiseinheit (2514), die in der Lage ist, die Antenne zu speisen,
wobei die Spaltringresonatorantenne an die Basiseinheit gekoppelt ist und wobei die Antenne Daten von der mindestens einen Steuerungseinheit drahtlos überträgt und empfängt, wobei das System die parasitären Effekte von dielektrischen Materialien in naher Umgebung zu einer tragbaren Funkfrequenzvorrichtung
minimiert.

3. Infusionspumpenbaugruppe nach Anspruch 2, ferner aufweisend eine Signalverarbeitungskomponente (2518).

4. Infusionspumpenbaugruppe nach Anspruch 3, wobei die Impedanzanpassungsschaltung (2526) neben der Signalverarbeitungskomponente (2518) integriert ist.

5. Infusionspumpenbaugruppe nach Anspruch 4, wobei die Signalverarbeitungskomponente(2518) ferner mindestens einen Filter aufweist.

6. Infusionspumpenbaugruppe nach Anspruch 4, wobei die Signalverarbeitungskomponente(2518) ferner mindestens einen Verstärker aufweist.

7. Infusionspumpenbaugruppe nach Anspruch 4, wobei die Signalverarbeitungskomponente(2518) ferner mindestens einen Schalter aufweist.

8. Infusionspumpenbaugruppe nach Anspruch 2, ferner aufweisend ein externes Infusionsset (134), das gestaltet ist, um das Infusionsfluid an einen Benutzer abzugeben.

9. Infusionspumpenbaugruppe nach Anspruch 1 oder 8, ferner aufweisend:
eine entsorgbare Gehäusebaugruppe (114) umfassend einen ersten Abschnitt eines Fluidabgabesystems und den Behälter und
eine wiederverwendbare Gehäusebaugruppe (102), umfassend einen zweiten Abschnitt des Fluidabgabesystems.

10. System nach Anspruch 1, wobei die Signalverarbeitungskomponente ferner mindestens einen Filter aufweist.

11. System nach Anspruch 1, wobei die Signalverarbeitungskomponente ferner mindestens einen Verstärker aufweist.

12. System nach Anspruch 1, wobei die Signalverarbeitungskomponente ferner mindestens einen Schalter aufweist.

## Revendications

1. Ensemble pompe à perfusion comprenant :
un réservoir (118) destiné à recevoir un fluide de perfusion ;
un ensemble pompe (106) afin de pomper une certaine quantité de fluide de perfusion du réservoir jusqu'à une sortie ;
un premier ensemble soupape (108) configuré de façon à isoler sélectivement l'ensemble pompe du réservoir ;
un second ensemble soupape (108) configuré de façon à isoler sélectivement la sortie de l'ensemble pompe ; et
une antenne à résonateur à bague fendue (2508) possédant une fréquence de résonance et comprenant une pluralité de couches métalliques planes,
ladite antenne à résonateur à bague fendue comprenant une bague extérieure (2502), une bague intérieure (2504), ladite bague intérieure (2504) étant entourée par la bague extérieure (2502) sans être en contact physique avec la bague extérieure, ladite bague intérieure possédant une fente (2506) et
un circuit d'adaptation d'impédance (2526), ledit circuit d'adaptation d'impédance étant intégré dans une ligne de transmission.

2. Ensemble pompe à perfusion selon la revendication 1, ledit ensemble pompe à perfusion comprenant en outre :
au moins une unité de régulation (2522) ; et
une unité de base d'émission/réception (2514) capable d'alimenter l'antenne, ladite antenne à résonateur à bague fendue étant couplée à l'unité de base et moyennant quoi l'antenne émet et reçoit, par l'intermédiaire d'une technologie sans fil, les données provenant de la au moins une unité de régulation, ce qui permet au système de minimiser les effets parasites de matériaux diélectriques se trouvant à proximité immédiate d'un dispositif de radiofréquence portable.

3. Ensemble pompe à perfusion selon la revendication 2 comprenant en outre un composant de traitement de signal (2518).

4. Ensemble pompe à perfusion selon la revendication 3, ledit circuit d'adaptation d'impédance (2526) étant intégré à proximité du composant de traitement de signal (2518).

5. Ensemble pompe à perfusion selon la revendication 4, le composant de traitement de signal (2518) comprenant en outre au moins un filtre.

6. Ensemble pompe à perfusion selon la revendication 4, le composant de traitement de signal (2518) comprenant en outre au moins un amplificateur.

7. Ensemble pompe à perfusion selon la revendication 4, le composant de traitement de signal (2518) comprenant en outre au moins un commutateur.

8. Ensemble pompe à perfusion selon la revendication 2 comprenant en outre un groupe de perfusion externe (134) conçu pour administrer le fluide de perfusion à un utilisateur.

9. Ensemble pompe à perfusion selon la revendication 1 ou 8, comprenant en outre :
un ensemble boîtier jetable (114) comprenant une première partie d'un système de distribution de fluide et le réservoir ; et
un ensemble boîtier réutilisable (102) comprenant une deuxième partie du système de distribution de fluide.

10. Système selon la revendication 1, ledit composant de traitement de signal comprenant en outre au moins un filtre.

11. Système selon la revendication 1, ledit composant de traitement de signal comprenant en outre au moins un amplificateur.

12. Système selon la revendication 1, ledit composant de traitement de signal comprenant en outre au moins un commutateur.
